# DEMANDE DE BREVET EUROPEEN

(11) **EP 0 682 244 A1**
(43) Date de publication de la demande: **15.11.1995**
(21) Numéro de dépôt: 95460023.5
(22) Date de dépôt: 10.05.1995
(51) Int. Cl.: G01N 21/25, C12M 1/34

(54) **Appareil de mesure pour le contrôle de la qualité bactériologique de l'eau**

(30) Priorité: 10.05.1994 FR 9405963
(71) Demandeur: GIE ANJOU-RECHERCHE, F-75008 Paris (FR)
(72) Inventeur: Santurette, Nadine, F-75012 Paris (FR); Rigaud, Gauthier, F-75012 Paris (FR)
(74) Mandataire: Vidon, Patrice

(57) **Abrégé**

L'invention concerne un appareil de mesure pour le contrôle de la qualité bactériologique des eaux caractérisé en ce qu'il comprend :
- au moins une enceinte thermostatée (1) permettant d'accueillir au moins une cuve de mesure (2) renfermant un échantillon d'eau auquel a été préalablement ajouté au moins un réactif,
- des moyens de mesure de l'absorption colorimétrique (3) dudit échantillon d'eau contenu dans ladite cuve de mesure,
- des moyens de mesure de la fluorescence (4) dudit échantillon d'eau contenu dans ladite cuve de mesure (2)
- des moyens d'enregistrement (5) permettant de mémoriser les données concernant ledit échantillon à tester mais aussi concernant lesdites mesures de l'absorption colorimétrique et de la fluorescence dudit échantillon, et,
- des moyens d'affichage (6) desdites mesures de l'absorption colorimétrique et de la fluorescence,

lesdits moyens de mesure de la fluorescence (4) étant destinés à être mis en oeuvre simultanément auxdits moyens de mesure de l'absorption colorimétrique (3) pour détecter la présence d'une contamination bactérienne dans ledit échantillon d'eau et son dénombrement.

## Description

L'invention concerne le domaine des analyses biologiques et le domaine du contrôle de la qualité des eaux.

La qualité des eaux de distribution est vérifiée régulièrement par un contrôle physico-chimique et par un contrôle bactériologique.

Les principaux objectifs du contrôle bactériologique sont essentiellement de garantir que l'eau présente une qualité conforme aux normes en vigueur permettant sa distribution, de renseigner sur l'évolution de la qualité bactériologique globale du réseau de distribution, et surtout de déceler le plus rapidement possible une éventuelle contamination fécale.

L'évaluation de la qualité bactériologique de l'eau repose en effet principalement sur la détection de la présence dans celle-ci de bactéries indicatrices de la pollution fécale. Les normes françaises actuellement en vigueur concernant ces bactéries sont les suivantes: absence de coliformes totaux et thermotolérants dans 100ml, absence de streptocoques fécaux dans 100ml et pas plus d'une spore de germes anaérobies sulfitoréducteurs dans 20 ml. Parmi ces catégories de germes, les coliformes totaux et thermotolérants sont presque toujours les germes fréquemment isolés de façon précoce lorsqu'une dégradation de la qualité bactériologique de l'eau est observée.

Ces bactéries coliformes sont exceptionnellement présentes en sortie des ouvrages de potabilisation mais des tests ont montré que les réseaux de distribution sont particulièrement exposés à une recontamination par ces germes, attribuable essentiellement à des phénomènes de croissance au sein des biofilms et au relargage de ces bactéries dans la phase eau. Ces épisodes sont surtout observés en période chaude, dans les zones des réseaux ou le résiduel de chlore est faible ou nul, et où la consommation élevée en matière organique biodégradable s'accompagne d'un dénombrement important de la flore totale planctonique.

Il existe de nombreuses méthodes de détection des bactéries coliformes dans les eaux.

La norme française actuellement homologuée (norme NF T 90-414) préconise d'effectuer une filtration de l'eau testée sur membrane et de déposer celle-ci sur un milieu de culture sélectif à base de gélose. Après une incubation de 24 h à 48 h à 37°C (ou à 44°C pour la recherche des coliformes thermotolérants) les colonies typiques ayant poussé sur la gélose sont dénombrées pour en déduire l'ampleur de la contamination.

Cette technique normalisée est couramment employée mais implique d'effectuer ensuite des tests de confirmation pour identifier précisément les bactéries d'après leurs caractères biochimiques (dégradation du lactose, absence de cytochrome oxydase, etc...).

La présence de bactéries coliformes dans l'eau n'est donc sûre qu'après une période d'au moins 48 heures ce qui représente un temps extrêmement long ne permettant pas d'optimiser les actions correctives sur le terrain pour la gestion du réseau de distribution au jour le jour. Ce délai peut se révéler particulièrement crucial en cas d'incidents sur le système de distribution d'eau ou en cas de pollution bactérienne provoquée par un agent susceptible d'être transmis par l'eau.

Donc, une telle méthode ne peut être automatisée de façon simple et réaliste.

Il a été proposé différentes méthodes visant à raccourcir le temps nécessaire à la détection dans l'eau de bactéries coliformes.

Parmi les techniques proposées, beaucoup sont basées sur une approche enzymatique. Ainsi, la demande de brevet française FR 2653888 décrit une méthode colorimétrique permettant de détecter semi-quantitativement des bactéries pathogènes dans l'eau. Cette méthode consiste à doser la quantité de nitrites présents dans un échantillon d'eau et résultant de la transformation des nitrates par action d'une enzyme bactérienne (la nitrate réductase), grâce à un réactif se colorant en présence de nitrites.

Une autre méthode, décrite dans la demande de brevet australien n°45317/79 consiste à utiliser la fluorescence d'une substance (telle que la di-β-D-galactopyranoside fluorescéine) lorsqu'elle se conjugue à une enzyme bactérienne (telle que la βgalactosidase).

Le brevet américain US 4,925,789 décrit une méthode enzymatique plus élaborée permettant de déceler relativement rapidement une éventuelle contamination fécale des eaux par des bactéries coliformes et plus particulièrement par *Escherichia coli(E. coli)*, bactérie thermotolérante archétype de la contamination fécale. Cette méthode peut être mise en oeuvre grâce à un test manuel constitué d'une poudre contenant deux réactifs:
- l'ortho-nitrophényl-galactoside (ONPG) destiné à être hydrolysé par l'enzyme β-galactosidase commune à toutes les bactéries coliformes , et
- le méthyl-umbelliféryl-glucuronide (MUG) destiné à être hydrolysé par l'enzyme β-glucuronidase, enzyme spécifique d'*Escherichia coli*, de *Salmonella*, et de quelques *Shigella*

Lors de la mise en oeuvre de cette méthode, la poudre est directement dissoute dans 100 ml d'échantillon à analyser. Après 24 heures d'incubation à 37°C, la présence des coliformes est mise en évidence par l'apparition d'une couleur jaune due au produit d'hydrolyse enzymatique, l'orthonitrophénol (ONP), et la présence d'*E.coli* est mise en évidence par l'apparition d'une fluorescence due à la formation d'un autre produit d'hydrolyse enzymatique, le méthyl-umbelliférone (MU). La présence d'une couleur jaune après incubation est évaluée visuellement par le laborantin et la présence d'une fluorescence est également évaluée de façon visuelle par celui-ci.

Cette technique présente principalement deux intérêts.

En premier lieu, elle permet la détection simultanée des coliformes totaux et de E.coli en une seule incubation d'une durée de 24H maximale et à la seule température de 37°C, par opposition aux autres méthodes.

En second lieu, elle autorise un délai de réponse en laboratoire plus court que les techniques classiques de culture sur gélose.

Toutefois, cette méthode présente aussi l'inconvénient d'être une méthode manuelle et donc de nécessiter une série de manipulations sur chaque échantillon testé. Elle ne peut donc être mise en oeuvre facilement sur un grand nombre d'échantillons, d'une façon économiquement intéressante.

De plus, l'appréciation tant de l'apparition de la coloration jaune que de la fluorescence ne peut être que subjective puisqu'effectuée visuellement par l'opérateur et par conséquent variant d'un opérateur à l'autre.

Enfin, si cette méthode permet de détecter la présence de bactéries coliformes et plus particulièrement d'*Escherichia coli* dans l'échantillon testé, elle ne permet pas d'évaluer efficacement à l'oeil nu la concentration en bactéries dans l'échantillon en fonction de l'intensité de la coloration ou de la fluorescence de l'échantillon testé.

L'objectif de l'invention est de fournir un appareil permettant d'automatiser au moins partiellement la lecture d'un test de contrôle de la qualité bactériologique de l'eau, mettant en oeuvre à la fois une coloration de l'eau testée et une fluorescence de celle-ci, quelles que soient les réactions mises en jeu.

Notamment, un objectif de l'invention est de proposer un appareil permettant d'automatiser la méthode décrite dans le brevet américain US 4,925,789 tout en raccourcissant les temps nécessaires à l'obtention de résultats exploitables.

Un autre objectif de l'invention est notamment de décrire un tel outil d'analyse automatique qui puisse être exploité directement par les laboratoires d'usine, et qui permette à travers un prélèvement manuel d'échantillons en divers points d'un réseau de distribution de l'eau, de mieux suivre l'évolution de la qualité de l'eau de ce réseau.

Encore un autre objectif de l'invention est de proposer un tel appareil ne nécessitant qu'un minimum d'opérations de la part d'un opérateur non spécialisé.

Encore un autre objectif de l'invention est de décrire un tel appareil permettant de raccourcir notablement le cycle : prise d'échantillons / analyses / interprétation / communication du résultat / action, qui est actuellement d'environ 3 à 5 jours avec les méthodes classiques normalisées à ce jour.

Encore un autre objectif de l'invention est de proposer un tel appareil permettant de détecter au plus tôt la croissance de la population bactérienne cible présente dans l'échantillon d'eau de façon automatique.

Un objectif de l'invention est également de pouvoir donner un résultat positif dès le dépassement d'un seuil fixé et paramétrable complètement indépendant de l'appréciation visuelle personnelle de l'opérateur, comme dans le cas des méthodes colorimétriques classiques, et d'abaisser ce seuil en-deçà du niveau perceptible à l'oeil nu grâce à des moyens adéquats.

Ces différents objectifs, ainsi que d'autres qui apparaîtront par la suite sont atteints grâce à l'invention qui concerne un appareil de mesure pour le contrôle de la qualité bactériologique des eaux caractérisé en ce qu'il comprend :
- au moins une enceinte thermostatée permettant d'accueillir au moins une cuve de mesure renfermant un échantillon d'eau auquel a été préalablement ajouté au moins un réactif,
- des moyens de mesure de l'absorption colorimétrique dudit échantillon d'eau contenu dans ladite cuve de mesure,
- des moyens de mesure de la fluorescence dudit échantillon d'eau contenu dans ladite cuve de mesure,
- des moyens d'enregistrement permettant de mémoriser les données concernant lesdites mesures de l'absorption colorimétrique et de la fluorescence dudit échantillon, et,
- des moyens d'affichage desdites mesures de l'absorption colorimétrique et de la fluorescence,

lesdits moyens de mesure de la fluorescence étant destinés à être mis en oeuvre simultanément auxdits moyens de mesure de l'absorption colorimétrique pour détecter la présence d'une contamination bactérienne dans ledit échantillon d'eau.

Un tel appareil intègre de façon particulièrement intéressante deux moyens de mesure optique permettant le contrôle de la qualité bactériologique d'une eau. La caractéristique consistant à effectuer la mesure de l'absorption colorimétrique et la mesure de la fluorescence sur un échantillon d'eau contenu dans une cuve de mesure sans avoir à changer l'échantillon de cuve entre les deux mesures, présente un intérêt particulier puisqu'il évite toute contamination croisée de l'échantillon lors de ces mesures, ou toutes pertes de bactéries lors du transfert d'échantillon ou du prélèvement aliquot. En effet, les fluorimètres et les spectrophotomètres disponibles sur le marché utilisent des cuves différentes.

De plus, les cuves ont classiquement un volume de quelques millilitres, ce qui oblige à travailler avec des fractions aliquotes, au détriment de la représentation réelle de la contamination de l'échantillon initial, de sa stérilité, de sa stabilité en température, et des longueurs de chemin optique trop courtes pour faciliter les détections en un temps suffisamment court. Les cuves préférentiellement utilisées auront un volume suffisant pour être représentatif et compatible avec les volumes préconisés dans les normes d'analyse bactérienne, soit 100 ml au minimum.

Dans l'hypothèse où l'on souhaite effectuer une mesure sur un fluorimètre puis une mesure de l'absorption colorimétrique sur un spectrophotomètre, il est alors nécessaire de changer l'échantillon de cuve. Malgré toutes les précautions qui peuvent être prises pour effectuer ce transfert, toute contamination de l'échantillon ne peut être écartée.

De plus, il n'existe pas actuellement sur le marché d'appareil permettant à la fois la mesure de la fluorescence et de l'absorption colorimétrique d'un échantillon et incluant de plus des moyens d'incubation des échantillons testés ce qui exclut tout suivi de la cinétique d'apparition de la fluorescence et de l'absorption colorimétrique à moins de replacer entre chaque mesure l'échantillon dans une étuve, ce qui est difficilement envisageable pour des mesures en grand nombre.

Préférentiellement, l'appareil selon l'invention comprend par ailleurs des moyens permettant de charger une pluralité de cuves de mesure contenant chacune un échantillon d'eau dans ladite enceinte thermostatée et des moyens permettant d'amener tour à tour chacune desdites cuves au niveau desdits moyens de mesure de l'absorption colorimétrique et desdits moyens de mesure de la fluorescence de l'échantillon. L'appareil permet ainsi de répondre à un souci de productivité et d'effectuer des mesures par salves sur un grand nombre d'échantillons pouvant provenir, par exemple, de différents points d'un réseau de distribution d'eau, de réservoirs, ou d'autres ressources en eau.

La présence d'une enceinte thermostatée au sein de l'appareil permet donc d'effectuer des mesures successives de ces deux caractéristiques sur un même échantillon et donc de pouvoir en déduire très précocement une contamination bactérienne et plus précisément la présence d'*E. coli* dans l'eau testée.

Un tel appareil est avantageusement mis en oeuvre pour automatiser le test décrit ci-dessus, le réactif utilisé étant alors, par exemple, constitué par de l'orthonitrophénylgalactoside et par du méthyl-umbelliféryl glucuronide. Toutefois, un tel appareil permet surtout d'optimiser une telle méthode puisqu'il autorise une détection plus rapide de l'apparition de la fluorescence et/ou de la coloration jaune de l'échantillon d'eau testé. On comprendra toutefois que l'appareil selon l'invention pourra être utilisé pour l'automatisation d'autres tests de contrôle de la qualité d'une eau, dès lors que cette méthode mettra en oeuvre une fluorescence et une coloration acquises simultanément ou à un très faible intervalle de temps au regard du phénomène d'évolution de l'échantillon examiné.

Egalement préférentiellement, l'appareil comprend des moyens d'introduction automatique dudit échantillon d'eau dans ladite cuve ou dans lesdites cuves.

Selon une variante de l'invention, il comprend également des moyens d'introduction automatique dudit réactif dans ladite cuve ou dans lesdites cuves. On notera toutefois que les cuves utilisées pourront aussi être des cuves dans lesquelles le réactif aura été introduit préalablement à l'ajout de l'échantillon liquide à examiner.

De tels moyens permettent d'automatiser les opérations manuelles nécessaires à la mise en oeuvre du test pratiqué pour évaluer la contamination bactériologique de l'eau testée sur un grand nombre d'échantillons avec la garantie d'une détection précoce.

D'une façon particulièrement intéressante, lesdits moyens de mesure de l'absorption colorimétrique et lesdits moyens de mesure de la fluorescence dudit échantillon d'eau comprennent avantageusement des moyens d'émission d'un rayon lumineux unique utilisé à la fois pour la mesure de l'absorption colorimétrique et la mesure de la fluorescence. Ainsi, le même faisceau lumineux est utilisé par les moyens de mesure de l'absorption colorimétrique et par les moyens de mesure de la fluorescence. D'une façon classique, les moyens destinés à mesurer l'absorption colorimétrique de l'échantillon peuvent comprendre une photodiode ou une barette de diodes et les moyens destinés à mesurer la fluorescence peuvent comprendre un tube photomultiplicateur.

On notera que, d'une façon classique la mesure de l'absorption colorimétrique se fait dans l'axe du rayon lumineux, tandis que la mesure de la fluorescence se fait préférentiellement à 90° par rapport à celui-ci.

Egalement avantageusement, lesdits moyens d'émission d'un rayon lumineux comprennent une source lumineuse disposée à l'intérieur d'une chambre pourvue d'une ouverture permettant le passage dudit rayon lumineux.

Selon une variante préférentielle, lesdits moyens permettant de charger une pluralité de cuves dans ladite enceinte comprennent au moins un plateau horizontal circulaire tournant autour de ladite chambre, ledit rayon lumineux étant essentiellement perpendiculaire auxdites faces desdites cuves. Un tel plateau rotatif permet le chargement d'un grand nombre de cuves qui peuvent défiler tour à tour devant le rayon lumineux afin de faire subir aux échantillons qu'elles contiennent une mesure de leur colorimétrie et de leur fluorescence.

Avantageusement, ledit plateau comprend une ouverture destinée à venir en regard avec au moins une partie du fond desdites cuves, de façon à permettre la mise en oeuvre desdits moyens de mesure de la fluorescence essentiellement perpendiculairement à la direction dudit rayon lumineux incident. La mesure de la fluorescence se fait ainsi grâce aux moyens de mesure de ce paramètre disposés au-dessous dudit plateau. L'ouverture en question peut également être mise en oeuvre de façon à permettre l'homogénéisation de la suspension contenue dans les cuves par agitation magnétique ou autre.

Selon une variante intéressante de l'invention, ledit plateau est constitué d'au moins deux anneaux concentriques pouvant être placés en rotation indépendamment l'un de l'autre, chacun desdits anneaux accueillant une série de cuves de mesure et au moins un espace libre permettant le passage dudit rayon lumineux, de façon telle, que celui-ci ne puisse passer qu'à travers une seule des cuves disposées sur ledit plateau afin de permettre la mise en oeuvre desdits moyens de mesure de l'absorption colorimétrique et de la fluorescence de l'échantillon d'eau contenu dans ladite cuve. De tels anneaux permettent l'analyse en parallèle d'un nombre important de cuves chargées dans l'appareil.

Selon une variante de l'invention, le système constitué d'une source, d'un détecteur fixe en ligne, et d'un détecteur orthogonal mobile dans le même plan, pourra être remplacé par le système constitué d'une source principale, d'un unique détecteur et d'un même nombre de sources orthogonales fixes que d'anneaux, celles-ci étant placées dans le même plan que la source principale et le détecteur.

Selon une variante de l'invention, au moins un emplacement du plateau peut être occupé par une cuve servant de référence active ou de référence passive. Une référence dite passive présentera des qualités d'absorption et de fluorescence prédéterminée. Une référence active et autonome sera constituée de diodes présentant un spectre et une intensité connue, dirigées vers les détecteurs d'absorption et de fluorescence.

Préférentiellement, chaque anneau présente au moins une ouverture permettant la mise en oeuvre des moyens de mesure de la fluorescence, c'est-à-dire le passage du faisceau lumineux à 90° par rapport aux moyens de mesure de la fluorescence, et/ou la mise en oeuvre de moyens d'homogénéisation de la suspension contenue dans les cuves.

Egalement selon une variante intéressante de l'invention, lesdits moyens de mesure de la fluorescence sont munis de moyens de translation leur permettant de coopérer avec au moins une ouverture de chacun desdits anneaux, selon un axe privilégié. On notera que l'on pourra également prévoir des moyens de mesure de la fluorescence fixes incluant autant de photomultiplicateurs que d'anneaux concentriques. Une telle solution est cependant moins intéressante économiquement.

Avantageusement, l'appareil selon l'invention inclut des cuves jetables après usage et des moyens permettant de stériliser celles-ci avant de les jeter.

Préférentiellement, lesdits moyens permettant de stériliser lesdites cuves de mesure incluent au moins une source U.V., ou des moyens d'injection de produits neutralisant la croissance bactérienne.

Par rapport à un fluorimètre ou à un spectrophotomètre classique, l'appareil présente là encore un autre avantage puisque de tels instruments de l'état de la technique ne sont classiquement pas pourvus de tels moyens, les cuves étant vidées et nettoyées une à une après que la mesure ait été effectuée, à l'aide d'un produit stérilisant.

Egalement préférentiellement, ledit appareil inclut des moyens d'entrée dans lesdits moyens de mémorisation d'une valeur seuil de l'absorption colorimétrique et d'une valeur seuil de fluorescence paramétrables. De tels moyens d'entrée pourront être constitués par un ordinateur ou un système équivalent dont la mémoire inclura lesdits moyens de mémorisation des mesures de coloration et de fluorimétrie.

Avantageusement, l'appareil comprend de plus des moyens d'alarme déclenchés lorsque les résultats desdites mesures de l'absorption colorimétrique et/ou de la fluorescence excèdent l'un et/ou l'autre desdits seuils. Ainsi, l'appareil selon l'invention pourra être utilisé pour surveiller la qualité de l'eau d'un réseau de distribution et alerter l'opérateur au plus tôt en cas de détection de bactéries coliformes et plus spécialement en cas de présence d'*Escherichia coli*.

L'invention concerne également une utilisation d'un tel appareil de mesure caractérisée en ce qu'elle consiste à effectuer dans le temps plusieurs mesures de l'absorption colorimétrique et de la fluorescence d'un échantillon d'eau contenu dans une cuve de mesure de façon à suivre l'évolution dans le temps de sa colorimétrie et de sa fluorescence et ainsi de détecter au plus vite la présence d'une contamination bactérienne, de la confirmer, et de pouvoir donner des réponses semi-quantitatives concernant la population initiale d'E.coli et/ou des coliformes totaux. La détermination de la population initiale se fera grâce à des tables statistiques incluant plusieurs variables (Absorption colorimétrique, fluorescence, temps) établies à partir de populations connues et quantifiées de germes et à plusieurs longueurs d'onde et pourra être effectuée en mettant en oeuvre automatiquement les corrections spectrales nécessaires comme celles dues à la turbidité progressive de l'échantillon.

L'invention, ainsi que les différents avantages qu'elle présente seront plus facilement compris grâce à la description de deux exemples non limitatifs de réalisation qui vont suivre en référence aux dessins dans lesquels :
- la figure 1 est une vue en coupe d'un premier mode de réalisation d'un appareil de mesure pour le contrôle de la qualité bactériologique des eaux selon l'invention, incluant un plateau rotatif ;
- la figure 2 est une vue schématique d'un second mode de réalisation d'un appareil selon l'invention incluant un plateau à trois anneaux concentriques ;
- la figure 3 est un graphique représentant l'évolution dans le temps de l'absorption entre 400 et 600 nm d'un échantillon d'eau auquel a été ajoutée un réactif, mesurée grâce à l'appareil représenté schématiquement à la figure 2 ;
- la figure 4 est un graphique représentant l'évolution dans le temps de la fluorescence dudit échantillon, après excitation à 366 nm.

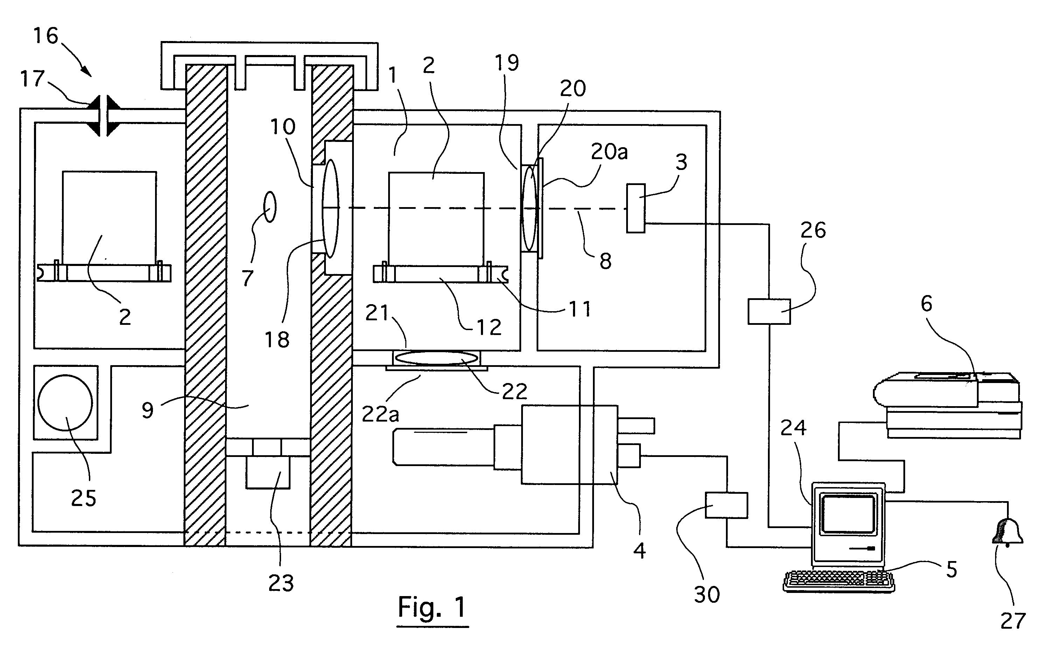

Selon la figure 1, l'appareil de mesure comprend une enceinte thermostatée 1 à 37°C destinée à accueillir une pluralité de cuves de mesure (2) fermées dans leur partie supérieure par un couvercle (2a) et renfermant chacune un échantillon d'eau dont on veut connaître la qualité bactériologique. Ces cuves (2), qui peuvent être jetées après usage contiennent déjà un réactif (décrit ci-après) et présentent avantageusement un couvercle pourvu d'au moins une zone susceptible d'être percée par une aiguille pour le remplissage desdites cuves, comme il sera explicité ci-après.

Les cuves (2) sont installées sur un plateau (11) circulaire rotatif présentant une pluralité d'ouvertures (12) sur lesquelles sont positionnées les cuves (2).

Des moyens automatiques de distribution (16) des échantillons d'eau et de réactif dans les cuves (2) sont prévus. Ces moyens de distribution de l'échantillon (16) se positionnent au dessus d'un passage (17) dans lequel peut être introduite une aiguille reliée à un système d'amenée de l'eau à tester et/ou de réactif. Lors de l'opération de remplissage d'une cuve, l'aiguille, préalablement stérilisée automatiquement, perce le couvercle de la cuve. Le mélange d'eau et/ou de réactif est ensuite amené dans celle-ci par la canalisation pour remplir la cuve. L'aiguille est ensuite retirée, stérilisée pour être utilisée pour remplir la cuve suivante.

Ces moyens de distribution permettent de normaliser le prélèvement des échantillons afin qu'ils présentent tous exactement la même quantité d'eau et la même quantité de réactif et ainsi de donner un caractère de grande reproductibilité au test effectué pour contrôler la qualité bactériologique de l'échantillon. De tels moyens permettent également de gagner du temps lors du remplissage des cuves et de permettre un tel remplissage dans de bonnes conditions de stérilité.

On notera que, dans d'autres modes de réalisation de l'invention, l'appareil pourra toutefois ne pas comprendre de tels moyens de distribution d'eau, le mélange de l'eau à tester et de réactif étant effectué dans les cuves manuellement avant de placer celles-ci dans l'appareil, sans pénaliser ses performances.

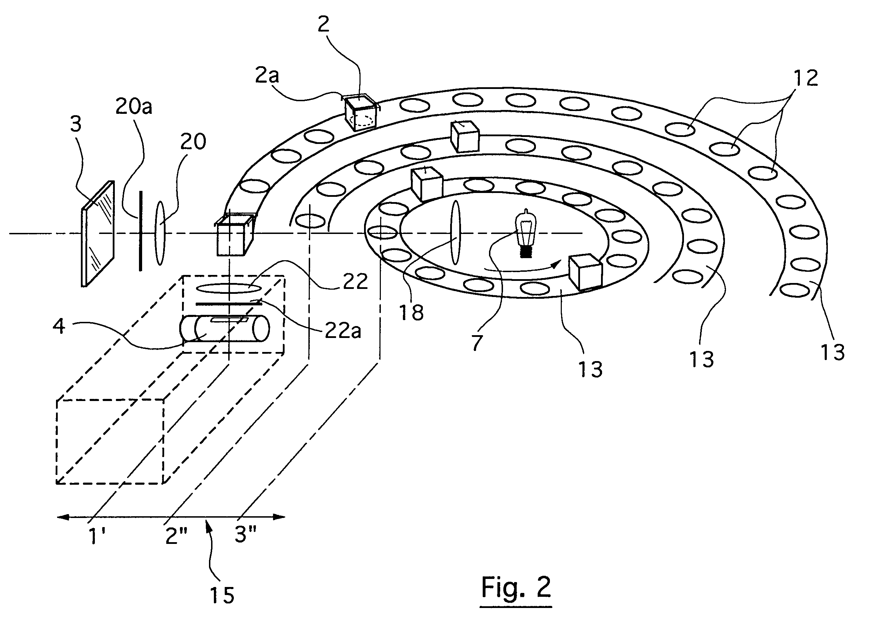

Dans le cadre du présent exemple de réalisation, ainsi que dans le cadre de l'exemple de réalisation décrit ci-après en référence aux figures 2 à 4, le réactif utilisé est une poudre contenant d'une part de l'ortho-nitrophényl-galactoside (ONPG) destiné à être hydrolysé par l'enzyme β-galactosidase commune à toutes les bactéries coliformes et à donner un produit de réaction présentant une coloration jaune, et d'autre part du méthylumbelliféryl-glucuronide (MUG) destiné à être hydrolysé par l'enzyme β-glucuronidase, enzyme spécifique d'*Escherichia coli* et à donner un produit de réaction fluorescent.

On notera que d'autres réactifs que ceux cités ci-dessus pourront être utilisés avec l'appareil selon l'invention dans le cadre de l'automatisation d'un autre test de contrôle de la qualité bactériologique d'une eau. A ce sujet, on notera également que l'enceinte pourra être thermostatée à une autre température que 37°C en fonction des micro-organismes dont on souhaitera détecter la présence. Il pourra notamment être envisager de thermostater l'enceinte à 44°C lorsque l'on souhaitera mettre en évidence la présence des germes thermotolérants, selon un procédé analogue.

Une chambre intérieure (9) essentiellement cylindrique est prévue sensiblement au centre de l'appareil pour accueillir une source lumineuse (7) constituée par une ampoule halogène. Cette chambre est pourvue d'une ouverture (10) dans laquelle est fixée une lentille colimatrice (18) permettant de créer un rayon lumineux (8) de référence, symbolisé sur la figure 1 par une ligne pointillée. Dans le but d'éviter une élévation de la température dans la chambre centrale (9) due à la présence de l'ampoule halogène, un ventilateur (23) est installée dans la partie inférieure de celle-ci . Un autre ventilateur (25) est également prévu sous le plateau tournant (11) pour homogénéiser la thermostation de l'enceinte.

L'appareil décrit comprend par ailleurs des moyens de mesure de l'absorption colorimétrique (3) de l'échantillon contenu dans les cuves (2) incluant une photodiode ou une barette de diodes ainsi que des moyens de mesure de la fluorescence (4) de cet échantillon incluant un photomultiplicateur. Ces moyens permettent de déterminer respectivement la coloration et la fluorescence de l'échantillon d'une des cuves présente sur le plateau (11), lorsque celle ci est amenée devant l'ouverture (10) comprenant la lentille colimatrice (18) et qu'elle est traversée par le rayon lumineux (8).

Conformément aux techniques classiques de spectrophotométrie, la mesure de l'absorption colorimétrique est effectuée dans l'axe du rayon lumineux et la mesure de la fluorescence de l'échantillon est effectuée à 90° par rapport à celui-ci. Afin de pouvoir effectuer la mesure de la colorimétrie, l'enceinte thermostatée 1 est munie d'une fenêtre (19) prévue dans l'axe du rayon lumineux (8) et équipée d'une lentille de focalisation (20) et d'un filtre (20a). De la même façon, afin de pouvoir effectuer la mesure de la fluorescence, l'enceinte thermostatée est munie d'une autre fenêtre (21), disposée essentiellement perpendiculairement au rayon lumineux (8) et pourvue d'une lentille de Fresnel (22).

L'appareil selon l'invention comprend également un ordinateur (24) incluant des moyens de mémorisation (5) permettant de mémoriser les mesures d'absorption colorimétrique et de fluorescence effectuées sur chaque échantillon par les moyens de mesure (3) et (4). Les signaux captés par ces éléments sont amplifiés grâce à des amplificateurs (30) et (26) et dirigés vers le micro-ordinateur (24).

Des moyens d'affichage (6) des mesures constitués par une imprimante graphique (pouvant notamment tracer des graphiques représentant les mesures sous forme de courbes) est connectée à ce micro-ordinateur (24). On notera que de tels moyens d'affichage des résultats de mesure pourront être aussi constitués par un écran graphique.

Des moyens d'alarme, symbolisés par la cloche (27) sont également connectés à ce micro-ordinateur.

Enfin, des moyens de stérilisation des cuves, également non représentés, et incluant au moins une lampe UV sont aussi prévus. Ces moyens peuvent être utilisés avant la mise en oeuvre des moyens de distribution des échantillons dans les cuves pour empêcher toute contamination bactérienne des cuves susceptible de biaiser les mesures. Ces moyens de stérilisation comprennent également des moyens de distribution d'un produit stérilisant, tel que de l'eau de Javel, dans les échantillons après que toutes les mesures aient été effectuées sur ceux-ci, avant de procéder au rebut de ces cuves.

Lors de l'utilisation de l'appareil décrit, le plateau tournant (11) amène automatiquement tour à tour chaque cuve (2) au niveau des moyens de distribution (16) afin de charger les échantillons dans celles-ci. Une salve d'échantillons préparés sur un portoir externe non représenté et provenant par exemple de différents points d'un réseau de distribution d'eau potable peuvent ainsi être chargés dans l'appareil très rapidement dans de bonnes conditions de stérilité. L'enceinte (1) étant thermostatée à 37°C, les bactéries éventuellement contenues dans les échantillons d'eau peuvent se multiplier et donc sécréter des enzymes réagissant avec les réactifs ONPG et MUG pour donner des produits de réaction détectables grâce à la photodiode et au photomultiplicateur.

Avant d'effectuer les mesures de l'absorption colorimétrique et de la fluorescence après incubation, les moyens de mesure (3) et (4) de ces deux paramètres sont mis en oeuvre pour effectuer une mesure de référence . Cette mesure peut être réalisée sur l'air. Une telle procédure permet de calibrer et de contrôler automatiquement la chaîne d'acquisition des mesures ponctuellement ou tout au long du cycle des mesures.

Les moyens de mesures de l'absorption colorimétrique et de la fluorescence sont ensuite mis en oeuvre sur un échantillon I d'eau avec réactif au temps t0 afin d'établir une valeur de référence temporelle pour l'échantillon I donné.

Après un temps t1 d'incubation, la coloration et la fluorescence de chaque échantillon sont mesurées et mémorisées par rapport à la valeur de référence absolue et à la valeur de référence temporelle. Puis, à intervalle de temps régulier, par exemple toutes les heures t2, t3..., les mesures sont de nouveau effectuées. La présence de bactéries coliformes et en particulier la présence d'E.coli, témoins d'une contamination fécale des eaux, peuvent ainsi être diagnostiquées très rapidement, les moyens de mesure de l'absorption colorimétrique et de la fluorescence de l'échantillon étant beaucoup plus sensibles et sélectifs pour telle ou telle longueur d'onde que l'oeil humain.

De plus, l'appareil selon l'invention peut être utilisé pour établir une gamme étalon de colorimétrie et une gamme étalon de fluorescence pouvant servir de bases à l'évaluation de la quantité de germes présents dans un volume d'eau donnée au moment du prélèvement. En colorimétrie et en fluorimétrie, les niveaux de densité optique pour chaque longueur d'onde permettent en effet, en fonction de leur cinétique d'évolution, de réaliser des évaluations de la quantité de germes initialement présents dans le volume d'échantillon, de façon plus représentative que les tables classiques dites MPN (most probable number) ou NPP (nombre le plus probable). La méthode en référence dans l'appareil présente le double avantage, pour la quantification, d'une détermination de la densité optique et de l'utilisation d'une cuve de mesure unique par échantillon (par opposition aux méthodes classiques NPP employant de multiples cellules de mesure pour un seul échantillon ). Dans le cadre de la présente invention, c'est l'intensité et la dynamique d'intensité de l'absorption colorimétrique et/ou de la fluorimétrie en fonction du temps, qui est évaluée pour établir une estimation du nombre de germes.

Le micro-ordinateur (24) peut être utilisé pour mémoriser une valeur seuil d'absorption colorimétrique et/ou une valeur seuil de fluorescence traduisant une présence de germes dépassant les normes de sécurité souhaitées. Dès que les mesures effectuées dépassent l'une ou l'autre de ces valeurs seuils, les moyens d'alarme (27) sont activés pour alerter l'opérateur.

En pratique, alors que les tests manuels classiques ne permettent l'obtention d'un résultat que dans un temps minimum de 24 heures environ, l'appareil selon l'invention permet de détecter la présence de bactéries coliformes dans une eau en un temps beaucoup plus court de l'ordre de 8 à 14 heures en fonction des niveaux de contamination initiaux.

De plus, il permet d'optimiser la méthode mise en oeuvre dans le test ONPG/MUG, en autorisant une évaluation quantitative approchée, et non plus seulement qualitative, de la contamination bactérienne. Les moyens d'alarme dont il est par ailleurs équipé permettent de plus d'attirer l'attention du laborantin chargé d'effectuer les tests qui peut alors très précocement prendre en compte l'existence d'une contamination bactérienne et l'origine préalablement saisie sur le micro-ordinateur de l'échantillon incriminé.

L'appareil représenté schématiquement à la figure (2) est sensiblement identique à celui représenté à la figure 1 sauf en ce qu'il comprend des moyens permettant de charger un nombre beaucoup plus important de cuves de mesures sur le plateau tournant. Pour les besoins de clarté de la description les pièces communes aux appareils représentés aux figures 1 et 2 portent les mêmes références.

Selon ce second mode de réalisation, le plateau tournant (11) est constitué de trois anneaux concentriques (13) installés dans le même plan horizontal et permettant d'accueillir chacun une pluralité de cuves de mesure (2) renfermant autant d'échantillons d'eau à tester. Chacun de ces anneaux est pourvu d'une pluralité d'ouvertures (12) correspondant à autant de places de cuves. (Pour clarifier la figure 2, seulement une ou deux cuves ont été représentées sur chaque anneau (13) et deux de ces anneaux ont été représentés partiellement.)

Afin de permettre la mise en oeuvre des moyens de mesure de l'absorption colorimétrique de l'échantillon sur une seule cuve à la fois, c'est-à-dire le passage du rayon lumineux (8) formée par la lampe (7) et la lentille (18) à travers une seule cuve à la fois, chacun des anneaux (13) peut tourner indépendamment des autres. En laissant un emplacement libre sur chacun des anneaux 13 en les faisant tourner séparément de façon à n'avoir qu'une seule cuve sur le trajet optique, il est ainsi possible d'atteindre le but recherché.

En ce qui concerne les moyens de mesure de la fluorescence (12) ceux-ci sont pourvus de moyens de translation (15) permettant d' amener le photomultiplicateur ainsi que le système optique (22, 22a) au-dessous de l'un ou de l'autre des anneaux (13) dans trois position correspondantes 1'', 2'' et 3''.

Après avoir examiné par exemple toutes les cuves du premier anneau, en effectuant une mesure d'absorption colorimétrique et de fluorescence simultanée ou quasi simultanée, les moyens de mesure de la fluorescence peuvent être déplacés sous le deuxième anneau,pour effectuer les mesures puis sous le troisième anneau. Les autres anneaux se positionnent de façon à ne pas intercepter le faisceau optique. Un tel système permet de prendre en charge initialement un plus grand nombre d'échantillons dans l'appareil et ainsi d'optimiser la productivité résultante. Celle-ci peut encore être améliorée avec un système basé sur une source centrale, une source sous chaque plateau et, dans le plan de la source principale, d'un détecteur unique, comme décrit précedemment.

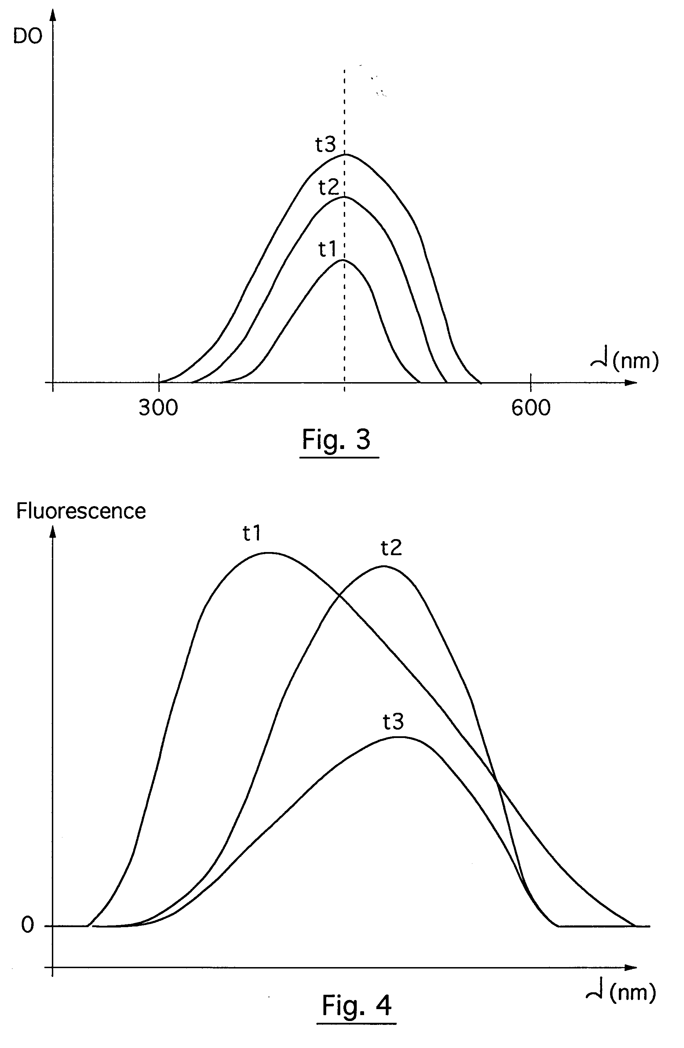

La figure 3 montre l'évolution dans le temps de l'absorption entre 400 et 600 nm d'un échantillon d'eau mesurée grâce à l'appareil représenté schématiquement à la figure 2, à différents temps t1, t2, t3 (t3>t2>t3). Au fur et à mesure des essais la coloration jaune de l'échantillon s'est intensifiée, traduisant la croissance bactérienne favorisée par l'incubation de l'échantillon au sein même de l'appareil selon l'invention. On notera que dans le cadre de l'utilisation de l'appareil selon l'invention avec d'autres réactifs que ONPG-MUG, l'absorption colorimétrique de l'échantillon pourra être lue dans une autre gamme de longueurs d'ondes que celles correspondant au jaune.

La figure 4 représente quant à elle trois spectres traduisant l'évolution dans le temps de la fluorescence du même échantillon après excitation à 366 nm. Ces spectres montrent des variations dans l'amplitude et la forme du spectre de fluorescence liées à la croissance bactérienne qui amène :
- une cinétique de dégradation des nutrients qui fluorescent (dans le cas de E.coli) et,
- une cinétique de dégradation de l'ONPG qui conduit à une intensification de l'accentuation de la coloration jaune qui se superpose et interfère avec le spectre de fluorescence.

Ces cinétiques respectives des différents phénomènes vont influencer les caractéristiques (la forme de l'intensité) et l'évolution du spectre de fluorescence final.

On notera que l'appareil selon l'invention pourra avantageusement être utilisé pour mettre en évidence certaines particularités d'évolution éventuellement non linéaires de la densité optique sur l'ensemble du spectre et permettre d'évaluer la population de telle ou telle espèce de bactérie.

Les exemples de réalisation de l'invention ici décrits n'ont pas pour objet de réduire la portée de l'invention. Ainsi, il pourra être envisagé d'apporter des modifications à ces exemples de réalisations sans sortir du cadre de celle-ci. On notera en particulier que les moyens de détection de la fluorescence et les moyens de détection de l'absorption colorimétrique de l'échantillon pourront être positionnés au sein de l'appareil de façon différente de celles indiquées.

## Revendications

1. Appareil de mesure pour le contrôle de la qualité bactériologique des eaux, caractérisé en ce qu'il comprend :
- au moins un plateau tournant horizontal essentiellement circulaire (11) sur lequel est chargée une pluralité de cuves de mesure (2) contenant chacune un échantillon d'eau ;
- une chambre (9) autour de laquelle est diposé le plateau (11) et à l'intérieur de laquelle est prévue une source lumineuse (7), ladite chambre étant pourvue d'une ouverture (10) permettant le passage du rayon lumineux (8) émis par ladite source (7) ;
- des moyens de mesure de l'absorption colorimérique (3) dudit échantillon ;
- des moyens de mesure de la fluorescence (4) dudit échantillon ;
- des moyens d'enregistrement (5) permettant de mémoriser les données concernant lesdites mesures de l'absorption colorimétrique et de la fluorescence dudit échantillon, et,
- des moyens d'affichage (6) desdites mesures de l'absorption colorimétrique et de la fluorescence,
lesdits moyens de mesure de la fluorescence (4) étant destinés à être mis en oeuvre simultanément auxdits moyens de mesure de l'absorption colorimétrique (3) pour détecter la présence d'une contamination bactérienne dans ledit échantillon d'eau, le plateau (11) présentant à cette fin au moins une ouverture (12) destinée à venir en regard avec au moins une partie du fond desdites cuves, de façon à permettre la mise en oeuvre desdits moyens de mesure de la fluorescence (4) essentiellement perrpendiculairement à la direction dudit rayon lumineux émis par la source lumineuse (7).

2. Appareil de mesure selon la revendication 1 caractérisé en ce qu'il comprend des moyens d'introduction automatique dudit échantillon d'eau dans ladite cuve.

3. Appareil de mesure selon l'une des revendications 1 ou 2 caractérisé en ce qu'il comprend des moyens d'introduction automatique d'au moins un réactif dans ladite cuve.

4. Appareil de mesure selon l'une des revendications 1 à 3 caractérisé en ce que ledit plateau est constitué d'au moins deux anneaux concentriques (13) pouvant être placés en rotation indépendamment l'un de l'autre, chacun desdits anneaux accueillant une série de cuves de mesure (2) et au moins un espace libre permettant le passage dudit rayon lumineux (8) de façon telle que celui-ci ne puisse passer qu'à travers une seule des cuves disposées sur ledit plateau afin de permettre la mise en oeuvre desdits moyens de mesure de l'absorption colorimétrique (3) et de la fluorescence (4) de l'échantillon d'eau contenu dans ladite cuve.

5. Appareil de mesure selon la revendication 4 caractérisé en ce que ledit plateau présente au moins un emplacement occupé par une cuve servant de référence active ou de référence passive.

6. Appareil de mesure selon les revendications 4 ou 5 caractérisé en ce que chaque anneau (13) présente au moins une ouverture (12) permettant la mise en oeuvre des moyens de mesure de la fluorescence (4).

7. Appareil de mesure selon la revendication 6 caractérisé en ce que lesdits moyens de mesure de la fluorescence (4) sont munis de moyens de translation leur permettant de coopérer avec au moins une ouverture de chacun desdits anneaux (12) selon un axe privilégié.

8. Appareil de mesure selon l'une des revendications 1 à 7 caractérisé en ce qu'il inclut des cuves jetables après usage et des moyens permettant de stériliser celles-ci avant de les jeter.

9. Appareil de mesure selon la revendication 8 caractérisé en ce que lesdits moyens permettant de stériliser lesdites cuves de mesure incluent au moins une source U.V. ou des moyens d'injection de produits neutralisant la croissance bactérienne.

10. Appareil de mesure selon l'une des revendications 1 à 9 caractérisé en ce qu'il inclut des moyens d'entrée dans lesdits moyens de mémorisation d'une valeur seuil d'absorption colorimétrique et d'une valeur seuil de fluorescence.

11. Appareil de mesure selon la revendication 10 caractérisé en ce qu'il comprend des moyens d'alarme déclenchés lorsque les résultats desdites mesures de l'absorption colorimétrique et/ou de la fluorescence excèdent l'un et/ou l'autre desdits seuils.

12. Utilisation d'un appareil de mesure selon l'une des revendication 1 à 11 caractérisée en ce qu'elle consiste à effectuer dans le temps plusieurs mesures de l'absorption colorimétrique et de la fluorescence d'un échantillon d'eau contenu dans une cuve de mesure de façon à suivre l'évolution dans le temps de sa coloration et de sa fluorescence et ainsi de détecter au plus vite la présence d'une contamination bactérienne, de la confirmer, et de pouvoir donner des réponses semi-quantitatives concernant la population initiale d'E.coli et/ou des coliformes totaux.
